# EUROPÄISCHE PATENTANMELDUNG

(11) **EP 2 151 776 A1**
(43) Veröffentlichungstag der Anmeldung: **10.02.2010**
(21) Anmeldenummer: 08021139.4
(22) Anmeldetag: 05.12.2008
(51) Int. Cl.: G06F 19/00

(54) **Vorrichtung zur Therapiebegleitung und zur Kontrolle von Medikationen beim Menschen**

(30) Priorität: 08.08.2008 DE 102008036940
(71) Anmelder: Medisana AG, 40724 Hilden (DE)
(72) Erfinder: Lindner, Ralf, 40597 Düsseldorf (DE)
(74) Vertreter: Ostriga, Sonnet, Wirths & Roche

(57) **Zusammenfassung**

Dargestellt und beschrieben ist eine Vorrichtung zur Therapiebegleitung und zur Kontrolle von Medikationen beim Menschen,

Aufgabe der Erfindung ist es, eine möglichst einfache, auch für ältere Menschen geeignete Vorrichtung zu schaffen, mit der diese zum Zwecke der Vertrauensbildung und der Erhöhung der Therapietreue zuverlässige Informationen über die ihnen verschriebenen Medikamente erhalten.

Die Lösung der Aufgabe wird dadurch gelöst, dass ein tragbares Gerät einschließlich eines Datenspeichers geschaffen wird, mit mindestens einer auf visuellen und/oder akustischen und/oder haptischen Informationen basierenden Kommunikationseinrichtung, wenigstens einer Bedieneinrichtung sowie einem Barcodescanner, wobei der Datenspeicher die Pharmazentralnummern-Strichcodes sämtlicher in Deutschland verfügbarer Medikamente sowie die Informationen der Beipackzettel beinhaltet.

## Beschreibung

Die Erfindung betrifft eine Vorrichtung zur Therapiebegleitung und zur Kontrolle von Medikationen beim Menschen.

Heutzutage wird im medizinischen Bereich immer mehr das Problem der Therapietreue (Non-Compliance) diskutiert, da man herausgefunden hat, dass ein hoher Prozentsatz von Patienten aufgrund schlechter Beratung und/oder mangelnden Vertrauens in die verordnete Therapie selbständig eingreifen, indem sie diese abwandeln oder vorzeitig beenden. Dabei ist immer wieder festzustellen, dass die Patienten über die sich daraus ergebenden Risiken nicht bewusst sind.

Grundsätzlich ist im hiesigen Gesundheitssystem zwar der Haus- oder Facharzt nicht nur für die Aufstellung des Therapieplanes, sondern auch für die Erläuterung zuständig. Jedoch ist dieser häufig aus Zeitdruck meist nicht in der Lage, dem Patienten die Therapie ausreichend zu erklären und zu begründen, so dass letztendlich diesem häufig auch das Vertrauen in die Therapie fehlt.

Bei vielen Patienten besteht auch das Problem der Vergesslichkeit, wobei dies viele Gründe, beispielsweise berufliche oder private Überlastung, Vorerkrankungen oder ein höheres Alter sowie Veranlagung sein kann.

Auch die Informationen seitens der Apotheken weisen häufig Mängel auf, wie jüngst noch Untersuchungen der Stiftung Warentest ergeben haben.

Aus dem Stand der Technik ist in diesem Zusammenhang allenfalls ein so genannter Medikamentenwecker bekannt, bei dem es sich gewöhnlich um ein diskusartiges Gerät mit im Zentrum angeordneter Programmiereinheit handelt, wobei um die Programmiereinheit herum sich kleine Fächer zur Einlage von Medikamenten befinden. Der Patient kann hier einprogrammieren, welches Medikament aus welchem Fach zu welcher Uhrzeit eingenommen werden soll.

Die Aufgabe der Erfindung besteht zunächst darin eine möglichst einfache, auch für ältere Menschen geeignete Vorrichtung zu schaffen, mit der diese zum Zwecke der Vertrauensbildung und der Erhöhung der Therapietreue zuverlässige Informationen über die ihnen verschriebenen Medikamente erhalten.

Die Lösung der Aufgabe ergibt sich aus den Merkmalen des Anspruches 1, insbesondere dem Kennzeichenteil, wonach es sich bei einer derartigen Vorrichtung um ein tragbares Gerät einschließlich eines Datenspeichers handelt, mit mindestens einer auf visuellen und/oder akustischen und/oder haptischen Informationen basierenden Kommunikationseinrichtung, wenigstens einer Bedieneinrichtung sowie einem Barcodescanner, wobei der Datenspeicher die Pharmazentralnummern-Strichcodes sämtlicher in Deutschland verfügbarer Medikamente sowie die Informationen der Beipackzettel beinhaltet.

Eine solche vorzugsweise in Handygröße ausgebildete Vorrichtung hat den grundsätzlichen Vorteil, dass sie dem Patienten auf sehr einfache Weise an jedem gewünschten Ort zuverlässige Informationen über die verschriebenen Medikamente verschafft. Der Patient muss lediglich das ihm vom Apotheker ausgehändigte Medikament mit dem Strichcode vor den Barcodescanner halten und hat dann die Möglichkeit, visuelle oder akustische Informationen über das verschriebene Medikament zu erhalten.

Vorzugsweise sollte die erfindungsgemäße Vorrichtung auch eine zweiseitige Kommunikationseinrichtung erhalten, die bei einer weiteren Ausführungsform auch die Möglichkeit eröffnet, dass die Kommunikationseinrichtung die vom Arzt festgelegte Medikation erfragt sowie alternative Medikationen mitteilt.

Eine weitere Ausführungsform der Erfindung weist eine Kommunikationseinrichtung auf, die auch auf eine visuelle und/oder akustische Einrichtung zur Erinnerung an die Einnahme von Medikamenten in fester, flüssiger oder gasförmiger Form erweitert ist.

Die vorher beschriebene Ausführungsform kann des Weiteren mit einer nach Art eines Medikamentenweckers ausgebildeten, mit einem zahlreichen Pillenfächer aufweisenden Zusatzbehälter gekoppelt sein, so dass nicht nur an die Medikamenteneinnahme erinnert, sondern der Patient zugleich auch akustisch oder visuell aufgefordert wird, gemäß der Therapie zusammengestellte Medikamente in einem bestimmten Pillenfach einzunehmen.

Darüber hinaus ist es bei einer weiteren Ausführungsform möglich, dass die Kommunikationseinrichtung eine visuelle und/oder akustische Einrichtung zur Erinnerung an Messungen von Körperparametern, wie Blutdruck, Temperatur od. dgl., aufweist.

Die vorgenannte Ausführungsform führt hin zu einer hinsichtlich der Funktion erweiterten Ausführungsform der Erfindung, bei der eine Einrichtung zur datenmäßigen Speicherung von Anzahl, Art und Zeitpunkt der Einnahme von gleichen bzw. unterschiedlichen Medikamenten vorhanden ist. Dadurch kann der Patient wie auch der Arzt über größere Zeiträume die Medikation oder auch die Therapietreue kontrollieren.

Die vorgenannte Ausführungsform ist jedoch auch Basis für eine sehr wesentliche, besonders bevorzugte zusätzliche Ausführungsform, bei der auch eine Einrichtung zur Überprüfung eventueller negativer Wechselwirkungen unterschiedlicher Medikamente sowie Kontraindikationen besteht.

Bei einer derartig bevorzugten Ausführungsform besteht des Weiteren die Möglichkeit, die Verordnungen von Medikamenten unterschiedlicher Ärzte sowie auch zusätzlicher, selbstständig beim Apotheker beschaffter rezeptfreier Medikamente auf gegebenenfalls vorhandene Wechselwirkungen bzw. Kontraindikationen automatisch zu untersuchen. Dies hat eine besonders große Bedeutung, da wissenschaftlich belegt ist, dass gerade bei älteren Patienten ein großer Prozentsatz der Einlieferungen in Krankenhäuser - vermutlich auch von Todesfällen - auf unerwünschte Wechselwirkungen völlig unterschiedlicher Medikamente beruht, die jedoch grundsätzlich bekannt sind.

Auf vorteilhafte Weise kann der Patient quasi selbsttätig und ohne großen Aufwand nach jeder hinzukommenden Verordnung eines Medikamentes mit dem erfindungsgemäßen Gerät Wechselwirkung und Kontraindikationen überprüfen.

Zur weiteren Verbesserung der erfindungsgemäßen Vorrichtung kann diese auch eine Einrichtung zur Speicherung gesundheitsrelevanter Daten sowie Vorerkrankungen aufweisen, wodurch die vorgenannten Überprüfungen seitens der Patienten mit Hilfe des erfindungsgemäßen Gerätes noch verbessert werden. Beispielsweise würde ein derartiges Gerät Alarm schlagen, wenn im Gerät Informationen vorliegen, dass eine Allergie auf Penizillin vorhanden ist, wenn jedoch trotzdem ein penizillinhaltiges Medikament verschrieben worden ist oder es würde ebenfalls zu einer Alarmmeldung kommen, wenn eine Vorerkrankung in Form eines Magen- oder Zwölffingerdarmgeschwürs vorhanden war und eine Verschreibung eines Medikamentes mit Acetylsalicylsäure erfolgt.

Bei einer weiteren bevorzugten Ausführungsform der Erfindung ist die Vorrichtung mit zusätzlichen Schnittstellen für medizinischtechnische Geräte, wie Blutdruckmessgerät, Blutzuckergerät od. dgl. gekoppelt, wodurch auf vorteilhafte Weise die von diesen Geräten erfassten Messwerte direkt mit eingespeichert werden könnten.

Die Anordnung einer weiteren Schnittstelle, die als Datentransferanschluss ausgebildet ist, ist darüber hinaus bei einer Ausführungsform denkbar. Dies hat den Vorteil, dass die erfindungsgemäße Vorrichtung beispielsweise über Internet jederzeit mit Updates hinsichtlich der in Deutschland vertriebenen Medikamente sowie auch mit neuen Informationen über Wechselwirkung von Medikamenten versehen werden kann.

Damit solche erfindungsgemäßen Geräte beispielsweise auch von Ehepaaren gemeinsam benutzt werden können, weist eine weitere erfindungsgemäße Vorrichtung eine Einrichtung zur Identifikation unterschiedlicher Menschen auf, die beispielsweise aus einem Fingerscanner gebildet sein kann. Dadurch ist es möglich, sofern zu Beginn der Benutzung des Gerätes die Identifikation erfolgte, dass beispielsweise Rentnerehepaare dieses Gerät gemeinsam nutzen können.

Weitere Vorteile der Erfindung ergeben sich aus der nachfolgenden Beschreibung eines Ausführungsbeispieles. Es zeigen:
- Fig. 1 die: skizzenhafte Darstellung der Grundeinheit einer Vorrichtung zur Therapiebegleitung und zur Kontrolle von Medikationen beim Menschen und
- Fig. 2: eine Vorrichtung nach Fig. 1 mit einer zusätzlichen Schnittstelleneinrichtung.

In den Zeichnungen ist eine Vorrichtung zur Therapiebegleitung und zur Kontrolle von Medikationen beim Menschen insgesamt mit der Bezugsziffer 10 bezeichnet.

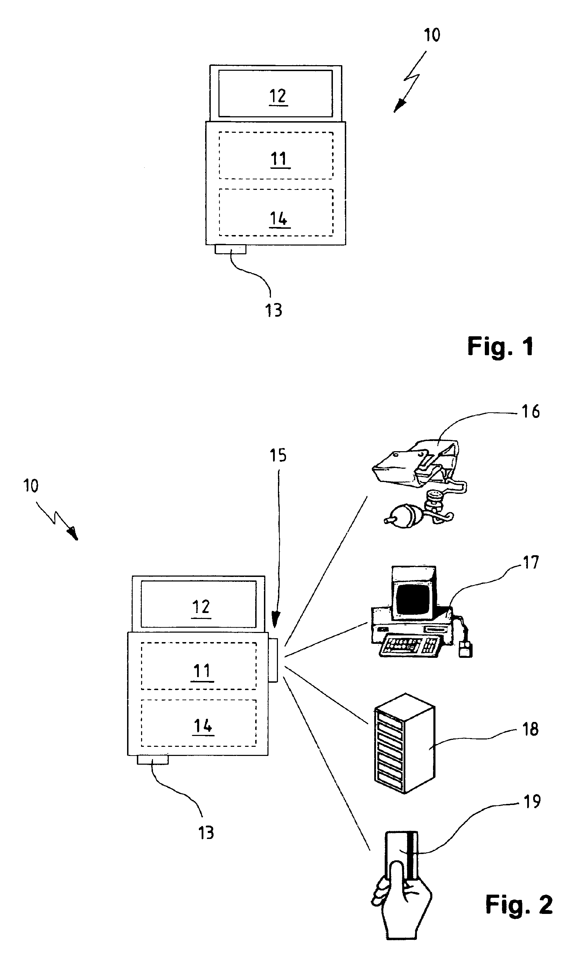

Eine derartige Vorrichtung 10 weist gemäß Fig. 1 einen Datenspeicher 11 auf, der die Pharmazentralnummern-Strichcodes sämtlicher in Deutschland verfügbarer Medikamente sowie die Informationen der Beipackzettel beinhaltet, einen Bildschirm 12 sowie einen Barcodescanner 13, wobei die Vorrichtung 10 über eine Bedieneinrichtung 14 steuerbar ist.

Die Vorrichtung 10 gemäß der Fig. 1, welche die Grundbaueinheit der erfindungsgemäßen Vorrichtung darstellt, kann erweitert werden durch eine Schnittstelleneinrichtung 15, welche beispielsweise Geräteanschlüsse 16, einen Datentransferanschluss 17, einen Anschluss 18 für eine Medikamentenspeichereinheit oder eine Identifikationseinrichtung 19 aufweist. Letztere kann womöglich aus einem Aufnahmeschlitz für eine Krankenkassenkarte bestehen, welche nach neuesten Entwicklungen auch Vorerkrankungen und Medikationen datenmäßig beinhaltet. Auch ist der Einsatz eines Fingerscanners denkbar.

Grundsätzlich wird darauf hingewiesen, dass die Bedienung der Vorrichtung, insbesondere für Sehbehinderte oder blinde Patienten, auch völlig ohne visuelle und stattdessen über akustische oder auch haptische Informationen geschehen kann.

Auch ist es denkbar, dass ein derartiges Gerät beispielsweise per Funk mit einem Arzt, Krankenhaus od. dgl. Kontakt aufnimmt, sofern lebensbedrohende Messwerte ermittelt werden.

Gegebenenfalls wäre es auch denkbar, dass dem Arzt automatisch Mitteilungen zugehen, aus denen sich die mangelnde Therapietreue oder eine irrtümlich fehlerhafte Medikation erkennen lässt.

## Patentansprüche

1. Vorrichtung zur Therapiebegleitung und zur Kontrolle von Medikationen beim Menschen, **gekennzeichnet durch** ein tragbares Gerät (10) einschließlich eines Datenspeichers (11), mindestens einer auf visuellen und/oder akustischen und/oder haptischen Informationen basierenden Kommunikationseinrichtung (12), wenigstens einer Bedieneinrichtung (14) sowie einem Barcodescanner (13), wobei der Datenspeicher (11) die Pharmazentralnummern-Strichcodes sämtlicher in Deutschland verfügbarer Medikamente sowie die Informationen der Beipackzettel beinhaltet.

2. Vorrichtung nach Anspruch 1, **gekennzeichnet durch** eine zweiseitige Kommunikationseinrichtung (12).

3. Vorrichtung nach Anspruch 2, **dadurch gekennzeichnet, dass** die Kommunikationseinrichtung (12) die vom Arzt festgelegte Medikation erfragt sowie alternative Medikationen mitteilt.

4. Vorrichtung nach einem der vorangehenden Ansprüche, **dadurch gekennzeichnet, dass** die Kommunikationseinrichtung (12) eine visuelle und/oder akustische Einrichtung zur Erinnerung an die Einnahme von Medikamenten in fester, flüssiger oder gasförmiger Form aufweist.

5. Vorrichtung nach Anspruch 4, **dadurch gekennzeichnet, dass** die Einrichtung mit einem zahlreiche Pillenfächer aufweisenden Zusatzbehälter (18) gekoppelt ist.

6. Vorrichtung nach einem der vorangehenden Ansprüche, **dadurch gekennzeichnet, dass** die Kommunikationseinrichtung (12) eine visuelle und/oder akustische Einrichtung zur Erinnerung an Messungen von Körperparametern, wie Blutdruck, Temperatur od. dgl., aufweist.

7. Vorrichtung nach einem der vorangehenden Ansprüche, **gekennzeichnet durch** eine Einrichtung zur datenmäßigen Speicherung von Anzahl, Art und Zeitpunkt der Einnahme von gleichen bzw. unterschiedlichen Medikamenten.

8. Vorrichtung nach einem der vorangehenden Ansprüche, **gekennzeichnet durch** eine Einrichtung zur Überprüfung eventueller negativer Wechselwirkungen unterschiedlicher Medikamente.

9. Vorrichtung nach einem der vorangehenden Ansprüche, **gekennzeichnet durch** eine Einrichtung zur Speicherung gesundheitsrelevanter Daten sowie Vorerkrankungen.

10. Vorrichtung nach einem der vorangehenden Ansprüche, **gekennzeichnet durch** zusätzliche Schnittstellen (16) für medizintechnische Geräte, wie Blutdruckmessgerät, Blutzuckermessgerät od. dgl..

11. Vorrichtung nach einem der vorangehenden Ansprüche, **gekennzeichnet durch** eine zusätzliche Schnittstelle, die als Datentransferanschluss (17) ausgebildet ist.

12. Vorrichtung nach einem der vorangehenden Ansprüche, **gekennzeichnet durch** eine Einrichtung (19) zur Identifikation unterschiedlicher Menschen.

13. Vorrichtung nach Anspruch 12, **dadurch gekennzeichnet, dass** die Einrichtung aus einem Fingerscanner gebildet wird.
